# EUROPEAN PATENT APPLICATION

(11) **EP 4 813 337 A1**
(43) Date of publication of application: **30.09.2026**
(21) Application number: 25224000.7
(22) Date of filing: 16.12.2025
(51) Int. Cl.: A61B 34/30, A61B 90/00, A61B 34/20

(54) **SYSTEM AND METHOD FOR POSITIONING A TOOL RELATIVE TO AN ANATOMICAL STRUCTURE USING FORCE CONTROL**

(30) Priority: 27.03.2025 WO PCT/EP2025/058422
(71) Applicant: Brainlab SE, 81829 München (DE)
(72) Inventor: TIRSCHLER, Thomas, 81829 München (DE); KRINNINGER, Maximilian, 81829 München (DE); EINSIEDLER, Christoph, 81829 München (DE)
(74) Representative: Eisenführ Speiser

(57) **Abstract**

The invention relates to a system for positioning a tool relative to an anatomical structure. The system comprises a robotic arm configured to position a tool and to adjust a force applied by the tool. The system also comprises force determination means to determine a force applied by the tool to the anatomical structure. A navigation system determines positions of a target tracker placed on the anatomical structure and of the tool. A controller receives data from the navigation system and the force determination means and determines whether the tool is in contact with the anatomical structure. After determining that the tool is in contact with the anatomical structure, the controller adjusts the force applied to the tool such that the magnitude of the force is within a predefined force range and the direction of the applied force is within a predefined range of directions.

## Description

The invention relates to a system and method for positioning a tool relative to an anatomical structure using force control. The tool may for instance be a drill guide that is positioned on a bone for directing a drill in an intended direction. The method involves the use of a robotic arm for holding and moving the tool. The robotic arm is further adapted to determine the force applied by the tool onto the anatomical structure and the robotic arm is controlled by controlling the force within predefined ranges.

The present invention pertains to the field of surgical equipment, with a particular focus on robotic systems utilized in surgical procedures. Specifically, it addresses the navigation of robotic arms during surgery, which is a critical aspect of modern surgical practice.

Robotic systems in surgery often include carrier structures, which may take the form of articulated support arms. These carrier structures can be subclassified into passive and active systems. Passive systems are primarily used to hold medical devices, appliances, and similar instruments in a desired spatial position, which includes both spatial location and orientation. These systems require manual alignment by a user, thus relying heavily on the precision and skill of the operator. Some comprise active brakes and other are generally friction based. An example of a passive system with active brakes for example is disclosed in WO 2016 075241 A1.

In contrast, active systems are equipped with actuators or motors, enabling them to align autonomously. Such systems can move the medical devices or appliances they are holding. To achieve autonomous alignment, active carrier structures may incorporate position encoders. These encoders provide data that allows for the calculation of the current spatial configuration of the carrier structure, sometimes also eliminating the need for external tracking systems that are often necessary for passive carrier structures lacking position encoders. A system with a driven robotic arm is disclosed in WO 2024 133824 A1.

Despite these advancements, there are notable limitations in the current state of robotic surgical systems. One of the primary challenges is the accuracy of navigation, which is essential for the success of any surgical procedure. Current systems often depend on tracking technologies to guide the movement of robotic arms. These tracking systems are susceptible to errors arising from various sources, such as sensor inaccuracies, signal interference, and calibration issues. Such errors can lead to deviations from the intended path of the robotic arm, compromising the precision of the surgical procedure.

Moreover, there is a significant issue concerning the movement between the tracked position of the robotic arm, a tool attached thereto and the actual anatomical target. This discrepancy can occur due to several factors, including the dynamic nature of the surgical environment, the flexibility of the anatomical target, patient movement, and the inherent limitations of the tracking technology. Consequently, there is a risk of misalignment between the robotic arm and the target area, which can adversely affect the outcome of the surgery. This is particularly the case when a tool held by the robotic arm is supposed to be held in a steady or controlled position with respect to the anatomical structure during surgery.

Additionally, the integration of robotic systems with existing surgical workflows presents further challenges. Achieving seamless coordination between the robotic system and the surgical team is critical, yet difficult with current technology. This lack of integration can lead to inefficiencies and increased operation times, further complicating the surgical process.

The limitations in the accuracy of navigation and the movement between the tracked position and the anatomical target underscore the need for improvements in robotic surgical systems. These challenges highlight the importance of developing more reliable and precise navigation technologies to enhance the performance of robotic arms.

In this context, it is an object of the invention to provide a system and method for positioning of a tool relative to an anatomical structure using a robotic arm to overcome the above limitations at least in part.

In a first aspect of the invention, the problem is solved by means of a system for positioning a tool relative to an anatomical structure. The system comprises a robotic arm which may comprise a plurality of segments driven by motors and an end-effector attached to the robotic arm and configured to hold the tool. The robotic arm is configured to position the tool and to adjust a force applied to the tool for instance by driving the motors. The robotic arm further comprises at least one force determination means to determine a force vector representing a magnitude and a direction of a force applied by the tool to the anatomical structure. The system may comprise a target tracker configured to be placed on the anatomical structure and a navigation system configured to generate a reference coordinate system and to determine positions of the target tracker and the tool in the reference coordinate system. Furthermore, the system comprises a controller configured to receive data like for example data indicating the positions of the target tracker and the tool from the navigation system and data indicating the force applied by the tool to the anatomical structure.

The controller may further be configured to determine from the received data whether the tool is in contact with the anatomical structure and control the robotic arm in accordance with the received data and the determination of a contact or no contact with the anatomical structure. The controller may further be configured to control the robotic arm to move the tool along a predefined trajectory relative to the position of the target tracker, when it is determined that the tool is not in contact with the anatomical structure, until it is determined that the tool is in contact with the anatomical structure. After determining that the tool is in contact with the anatomical structure, the controller may be configured to adjust the force applied to the tool such that the magnitude of the force is within a predefined force range and the direction of the applied force is within a predefined range of directions.

The invention is based on the idea that once contact is made between the anatomical structure, e.g. a bone of a patient, and the tool, e.g. a drill guide, the positioning of the tool can be controlled primarily based on the force that is applied to the anatomical structure by the tool. Using the force rather than the position determined by the navigation system improves the reaction time of the system to movements of the anatomical structure because the tool automatically follows the anatomical structure by always applying a certain amount of force on the structure. In addition, the accuracy of the tool following the anatomical structure is improved.

When using force controlled positioning in accordance with the invention, the robotic arm can determine the force that it exerts on an anatomical structure by itself and may react accordingly. It does not rely on an external optical navigation system. Force controlled positioning can be significantly quicker in reacting to changes than a navigation based on an optical navigation system. The robotic arm may for instance determine the force at a rate of 1 kHz, which is higher than the typical rates of optical navigation systems. In addition, optical navigation systems require a free line of sight. The force controlled navigation does not have this limitation. Force controlled navigation can therefore be applied in situations where the surgical team or further equipment is required to be close to the target structure and eventually blocks the line of sight of an optical navigation system.

The robotic arm may be any mechanical device designed to emulate the functions and movements of a human arm. The segments of the robotic arm are structural components that are connected in a series or sequence, allowing for articulated movement. Each segment is typically connected to the respective adjacent segment by a joint, and the movement of these segments is controlled by motors. These motors can be electric, hydraulic, or pneumatic, depending on the design and application of the robotic arm. The motors are preferably placed within the joints or may be connected to the joints by a mechanical transmission mechanism. In a surgical robotic arm, the segments of the robotic arm may be designed to mimic the movement of a human arm, with joints that allow for rotation and bending, enabling precise positioning of surgical instruments.

The end-effector is a critical component of the robotic arm, serving as the interface between the robot and the task it performs. It is attached to the distal end of the robotic arm and is specifically configured to hold and manipulate a tool. The tool attached to the end-effector can take various forms depending on the application; in a surgical context, it might be a drill guide, a gripper, a welding torch, or a scalpel holder. The design of the end-effector and its compatibility with specific tools is crucial as it determines the functionality and versatility of the robotic arm in performing specific tasks. In a surgical setting, the end-effector must be capable of securely holding delicate instruments while allowing for precise control and manipulation.

The robotic arm is able to not only place the tool in a desired location but also to control the amount of force exerted by the tool. For example, the arm must be able to position a tool with extreme accuracy and apply just the right amount of pressure to perform the intended task without damaging surrounding tissues. The motors play a crucial role in this process, as they provide the power and control needed to achieve the desired positioning and force adjustments, ensuring that the robotic arm can perform its tasks effectively and safely. For example, torque-controlled drives in each robot axis can be used to control the resulting force on the end-effector.

The navigation system is designed to track and guide the movement of objects or instruments with high precision. The navigation systems can employ various types of sensors and technologies to achieve accurate navigation and control. Common types of navigation systems include optical, electromagnetic, ultrasonic systems and position encoders of robotic arms. Also a combination of these technologies may be used in one navigation system. The core functionality of these systems involves capturing real-time or near real-time data from the environment or the objects themselves, processing this data through specialized software, and providing feedback to guide the movement and positioning of the objects. An optical navigation system may or may not use light signals in the visible spectrum. The cameras may capture real-time or near real-time images or video, and the data is processed to determine the exact position and orientation of the tracked objects in three-dimensional space. This type of system is particularly valuable in medical and surgical applications, where it can guide surgical instruments or robotic arms with high precision relative to the patient's anatomy.

Trackers are objects that are particularly designed for being tracked by the navigation system. Depending on the technology used by the navigation system, a tracker may comprise reflective markers, a recognizable pattern, or active signal generators. The navigation system may for instance be an optical navigation system utilizing optical sensors and cameras to track objects equipped with reflective markers, LEDs, infrared reflectors or the like. A target tracker is understood as a tracker that is configured to be attached to the anatomical target. The target tracker may comprise a screw configured to be temporarily attached to a bone. Using the screw, the tracker can be rigidly fixed to the bone, such that the position of the target tracker is representative of the position of at least this bone to which it is screwed. In addition, surrounding anatomical structures may be inferred to move with the target tracker. As an example, in spine surgery, a target tracker may be connected to a vertebral of the spine. The position of the tracker then directly indicates the position of this vertebra. In order, to leave room for the surgeon, it may be advantageous to attach the tracker not to the vertebra on which the surgeon wants to operate. Instead, the tracker may be attached two to three vertebrae further from the target vertebra. In this case, the position of the tracker is still indicative of the position of the target. For instance, overall movements of the patient are well represented by the target tracker. However, due to the flexibility of the spine, the target may move by a certain degree relative to the target tracker.

The navigation system generates a reference coordinate system in which each of the tracked objects is placed. The reference coordinate system can be a reference frame that is a rest frame of a part of the navigation system. For instance, a reference coordinate system may be chosen such that sensors of a navigation system (e.g. cameras) are at rest in the coordinate system. Typically, it is only required to track relative positions between the surgical equipment and the anatomical target or target trackers attached thereto. Therefore, the reference coordinate system may also change within an operating room, for instance, when a part of the navigation system is moved.

The controller is preferably configured to manage and direct the operations of a robotic arm, ensuring precise and efficient movement and functionality. The controller is able to follow algorithms and is equipped with the corresponding processing capabilities. The controller may interpret input signals from various sensors and user interfaces. It processes these inputs to generate appropriate control signals that drive the actuators of the robotic arm, enabling it to perform complex tasks with high accuracy. The controller can also be designed to adapt to real-time or near real-time feedback, allowing for dynamic adjustments to the robotic arm's movements. "Real-time or near real-time" in the context of this application refers to an action that is taking place with at least with a frequency of 10 Hz and delay of less than 0.1s. In certain cases, real-time navigation systems and controllers achieve a frequency of at least 250 Hz or at least 500 Hz.

The data received from the navigation system may be received from a central unit of the navigation system or from individual components of the navigation system and subsequently be combined in the controller of the robotic arm. The data indicative of the position of the robotic arm and the tool attached thereto may be received from the robotic arm itself and/or from separate sensors/trackers of the navigation system. Data indicating the force applied by the tool to the anatomical structure may be related to torque controlled motors in the joints of the robotic arms. The received data may be pre-processed to directly indicate the respective positions and the applied force on the anatomical structure or the controller may be designed to determine the respective positons. The controller may comprise one or more interfaces for receiving the respective data. The interfaces may be wired or wireless interfaces.

The data indicating the force applied by the tool to the anatomical structure may be generated based on measurement by the torque-controlled motors in the joints of the robotic arm. The generation may involve the use of a kinematic model of the robotic arm taking into account gravity and the positioning of the robotic arm. Additional sensor data, may also be included in the data indicating the force applied by the tool. For instance, dedicated sensors may provide an indication that the tool has established contact with the anatomical structure. Such sensors may be mechanical, optical or electrical sensors providing information on the contact between the tool and the anatomical structure.

The steps of the control algorithm, may be performed just once or repeatedly. Additional steps, may be performed between the steps described above. Furthermore, the order in which the steps are carried out is not fixed but may be adjusted for a specific application. Specific applications, may involve placing the tool on different anatomical structures one after another. It may also be considered that the arm may be guided into contact with the anatomical structure by a surgeon and only follows the predefined trajectory when contact is lost.

In an aspect currently claimed in claim 1, adjusting the force applied to the tool involves adjusting the position of the tool and following the anatomical structure when the anatomical structure moves in any direction. In one or more other aspects of the present disclosure, these features are omitted, i.e. these features are optional in this disclosure.

When the adjustment of the force involves adjusting the position of the tool and following the anatomical structure when the anatomical structure moves in any direction, this has the advantage that the positioning of the tool is primarily force controlled and allows to follow the anatomical structure even if the anatomical structure moves during the process of positioning the tool. Thus, the tool follows the anatomical structure when the anatomical structure gives way to the applied force or the tool is pushed back by the anatomical structure if the counterforce exceeds the intended force applied by the tool. Movement of the anatomical structure may involve translation and/or rotation of the anatomical structure. Using the force controlled positioning of the tool, the tool can follow the anatomical structure during translations and rotations. This embodiment allows to mimic the actions of a human surgeon holding the tool in position on the anatomical structure.

In some embodiments, the controller is further configured to, after determining that the tool is in contact with the anatomical structure, adjust the position of the tool using the robotic arm such that the position of the end-effector relative to the target tracker does not deviate more than a predefined maximal distance from the predefined trajectory. Adjusting the position in this way has the advantage that another parameter is controlled during positioning of the tool relative to the anatomical structure. This may for instance prevent a misplacement of the tool due to slipping of the tool along the anatomical structure. A positioning that is exclusively force controlled has the disadvantage that the relative position between the tool and the anatomical structure may change without affecting the force applied by the tool on the anatomical structure. For instance, a drill guide may slide along a bone surface, away from the predefined trajectory while applying a constant force to the bone. This is prevented by ensuring that the position of the end-effector relative to the target tracker does not deviate more than a predefined distance from the predefined trajectory. The predefined distance should be large enough such that a sufficient amount of flexibility of the anatomical structure is allowed within the tolerance of the predefined maximal distance. Such a small deviation is not to be interpreted as a misplacement of the tool but only as the anatomical structure giving way to the exerted force by a certain amount. At the same time, the predefined maximal distance should be chosen small enough such that, when the position of the tool deviates by a distance larger to what can be explained by the flexibility of the anatomical structure, the tool is repositioned closer to the predefined trajectory. Therefore, this embodiment includes an additional safety ensuring correct placement of the tool after a contact has been established.

In another embodiment, the controller is configured to determine whether the tool is in contact with the anatomical structure based on the data indicating the force applied by the tool to the anatomical structure. When the tool is not in contact with the anatomical structure it may not apply any force to the structure. Therefore, any applied force that is measured by the force determination means may be interpreted as an indication of a contact between the tool and the anatomical structure. Nonetheless, other data may be taken into account in the determination of a contact. For instance, if the distance between the target tracker and the tool is unexpectedly large when a force is applied, it may be determined that the tool is in contact with an obstacle rather than the target anatomical structure. This embodiment has the advantage that no additional sensor is required for determining a contact between the anatomical structure and the tool.

A further embodiment provides that the controller is configured to determine whether the tool is in contact with the anatomical structure by determining if the robotic arm can apply a force to the tool without moving the tool in the corresponding direction. When the tool is not in contact with the anatomical structure, the only forces acting on the tool are gravity and the force applied by the robotic arm. Since the robotic arm compensates for the gravitational force by holding the tool, any additional force applied by the arm will result in a movement of the tool in this situation. If the tool gets into contact with the anatomical structure, the anatomical structure may provide a counterforce stopping this movement. The described embodiment uses this as a sign for determining a contact between the tool and the anatomical structure, which has the advantage that a tactile determination of contact is provided similar to the one that a surgeon would use when handling the tool.

In a related embodiment, the controller is further configured to, upon determining that the tool is in contact with the anatomical structure, record an initial point of contact which corresponds to the position of the tool relative to the target tracker at a moment of first contact. The controller determines a deformation of the anatomical structure by determining that by adjusting the force applied to the tool, the position of the end-effector relative to the target tracker has changed from the initial position of contact. It is important to distinguish, in this context, between a displacement of the target and a deformation of the target. Displacement refers to any movement of the target from its initial position. It can for instance be caused by a movement of the patient or the operating table. Deformation refers to a change in the relative position between the target tracker (which is connected close to the point of contact but not directly thereon) and the point of contact. The initial point of contact may be recorded even before the force applied by the tool is within the predefined force range. Thus, the initial point of contact represents the point of contact relative to the target tracker without deformation caused by the tool. In the example of a target tracker attached to a vertebra close to a target vertebra, the initial point of contact represents the position of the target vertebra before applying a force. In order to achieve a stable position of the tool, a force within the predefined force range is applied. This may cause the target vertebra to give way by a certain amount to the applied force. The mentioned embodiment above is able to detect such a movement and identify it as a deformation of the anatomical structure.

In another related embodiment, the controller is further configured to determine the flexibility and/or elasticity of the anatomical structure based on the force applied to the anatomical structure and the deformation of the anatomical structure. This has the advantage that the system determines information on the anatomical structure which may be fed into an anatomical model and be used for target tracking or other purposes. Using this data, the anatomical model or an individual patient model, can be constantly updated and improved. Thanks to its palpation capabilities, the system can also enable Al-supported optimization of robot-assisted tasks, such as positioning and tracking. The flexibility and/or elasticity may in particular be measured from a curve showing the deformation of the target as a function of the force applied by the tool on the anatomical target.

In another embodiment, the predefined trajectory, the force range and the range of directions can be set by a user of the system. If applicable, the user may also set the predefined maximal distance from the predefined trajectory. This, embodiment has the advantage that the mentioned parameters may be set in accordance with the requirements of the task. For instance, tracking a firm anatomical structure like a bone may be done by applying a relatively strong force by the tool, whereas a softer anatomical structure may only withstand a weaker force by the tool. Also the acceptable range of directions depends on the task carried out during an operation and specifically on the precision requirements, which can for instance be dictated by sensitive neighboring anatomical structures that have to be protected.

The controller may also perform a plausibility check with a patient model. The plausibility check may allow to determine whether the combination of deformation of the anatomical structure and application of force is in line with an expected amount of deformation based on an expected elasticity of the anatomical structure. If the plausibility check is positive, the tracking and compensation is continued, otherwise it is suspended. For example, it is possible to determine whether the vertebral body is behaving as expected, i.e. whether the deformation corresponds to a corresponding amount of force that matches the stored empirical values how much a vertebra is expected to give way to the applied force.

In another aspect of the disclosure, a method for positioning a tool relative to an anatomical structure is disclosed. The method is carried out by a controller of the robotic arm, for instance by a controller like the one included in the system according to the first aspect of the invention. The controller carries out or controls the respective components of the system to perform the steps described above in relation to the first aspect of the invention.

The same method may also be used for positioning a tool relative to an object, wherein the object may or may not be an anatomical structure. In particular, the object may not be part of a human or animal body. The object may instead be a polymer test body, a work piece in a production process or an object held by a user during cooperative work with the robotic arm. The method may also be used for positioning a tool relative to an anatomical structure that is not part of a living human or animal body and is neither returned thereto. In some embodiments, the method is used for placing the tool on the anatomical structure in a non-invasive manner. The method may for instance be used for supporting non-medical and non-invasive cosmetic procedures like tattooing, piercing, hair removal by optical radiation or micro-abrasion of the skin.

In another aspect of the invention, a method of determining control instructions for a robotic arm is disclosed. The robotic arm comprises a plurality of segments connected by joints and driven by motors, and the control instructions comprise computer-readable commands for the robotic arm to adjust a pose of the robotic arm and/or a torque generated by at least one of the motors acting in at least one of the joints. The method comprises receiving data indicating the position of a target tracker and a position of a tool held by the robotic arm; receiving, from a force determination means of the robotic arm, data indicating a force, wherein the data indicates a force vector representing a magnitude and a direction of a force acting on the tool. The method further comprises determining from the received data whether the tool is in contact with an object; and determining control instructions for the robotic arm based on the received data and the determination of a contact or no contact with the object. Determining control instructions for the robotic arm comprises, when it is determined that the tool is not in contact with the object, generating control instructions to adjust the pose of the robotic arm to move the tool along a predefined trajectory relative to the position of the target tracker until it is determined that the tool is in contact with the object; and after determining that the tool is in contact with the object, generating control instructions to adjust the torque by at least one of the motor acting in at least one of the joint such that the magnitude of the force is within a predefined force range and the direction of the force is within a predefined range of directions.

In an aspect of the method currently claimed in claim 8, the adjustment of the torque by the at least one motor acting in at least one of the joints is also such that the tool follows the object when the object moves in any direction. In another aspect of the present disclosure, these features are omitted from the method.

In a third aspect of the invention, a robotic arm for positioning a tool relative to an anatomical structure is disclosed. The robotic arm is in accordance with the robotic arm included in the first aspect of the invention. In this aspect, a controller is included in the robotic arm and causes the robotic arm to perform the respective method steps of the second aspect of the invention.

For a robotic arm as currently claimed in claim 13, the adjustment of the force applied to the tool involves adjusting the position of the tool and following the anatomical structure when the anatomical structure moves in any direction. In another aspect of the present disclosure, these features are omitted from the robotic arm.

In a fourth aspect of the invention, the disclosure relates to a computer program product. The computer program product comprises instructions to cause a system in accordance with the first aspect of the invention to execute the steps included in the method of the second aspect of the invention.

For a more complete understanding of the invention, the invention will now be described in detail with reference to the accompanying drawings. The detailed description will illustrate and describe what is considered as a preferred embodiment of the invention. It should of course be understood that various modifications and changes in form or detail could readily be made without departing from the spirit of the invention. It is therefore intended that the invention may not be limited to the exact form and detail shown and described herein, nor to anything less than the whole of the invention disclosed herein and as claimed herein after. Further, the features described in the description, the drawings and the claims disclosing the invention may be essential for the invention considered alone or in combination. In particular, any reference signs in the claims shall not be construed as limiting the scope of the invention. The wording "comprising" does not exclude other elements or steps. The word "a" or "an" does not exclude the plurality. The wording "a number of" items comprising also the number 1, i.e. a single item, and further numbers like 2, 3, 4 and so forth. In the accompanying drawings:
Fig. 1 shows an exemplary and illustrative drawing of a system for positioning a tool relative to an anatomical structure inside an operating room;
Fig. 2 shows an schematic drawing of the system for positioning a tool and its components;
Fig. 3 shows an exemplary and illustrative drawing of a tool positioned on an anatomical structure;
Fig. 4 is an illustration of the acceptable ranges of the force applied by the tool on the anatomical structure, resulting in a force cone or conical frustum;
Fig. 5 illustrates the tracking of an anatomical structure with the tool using force-controlled positioning when the anatomical structure moves by translation without being rotated;
Fig. 6 illustrates the tracking of an anatomical structure with the tool using force-controlled positioning when the anatomical structure moves by translation and rotation;
Fig. 7 illustrates the case in which the tool does not make contact with the anatomical structure in the expected position; and
Fig. 8 is an exemplary flow chart of a method for positioning a toll relative to an anatomical structure.

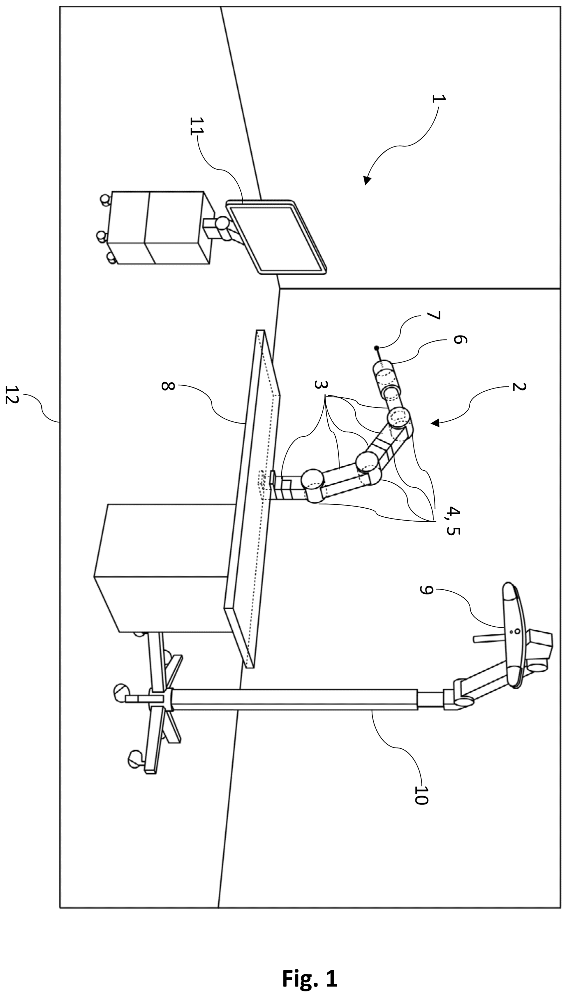

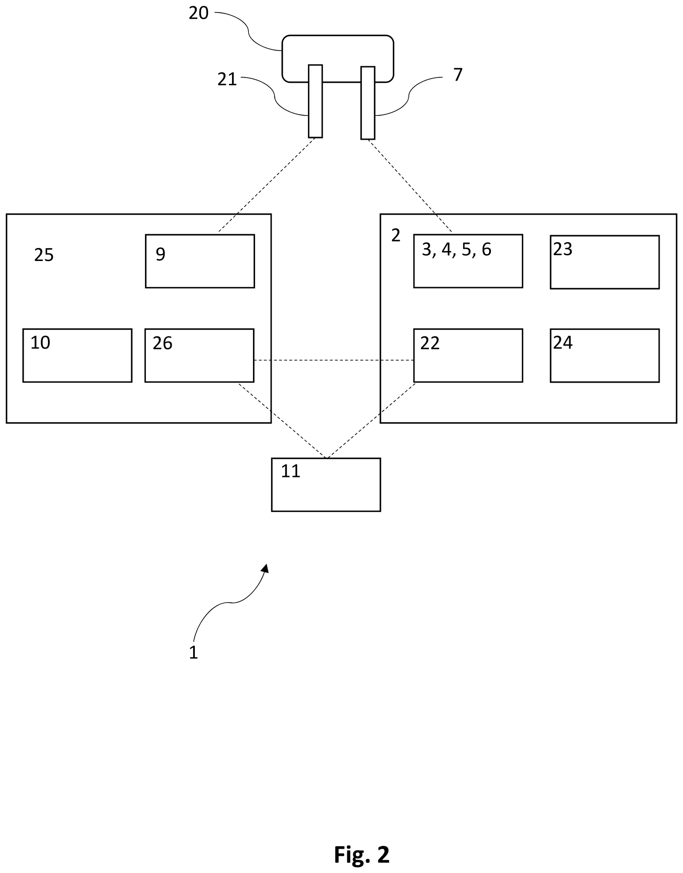

Fig. 1 shows a system 1 for positioning a tool 7 relative to an anatomical structure 20 (see Fig. 2). Such a system 1 can for instance be used for surgery. Fig. 1 shows the system 1 in an operating room 12 having an operating table 8 in its center. The operating table 8 is configured to carry a patient (not shown) and position the patient within the range of the robotic arm 2 of the system 1. In the shown embodiment, the robotic arm 2 is directly attached to the operating table 8. This has the advantage that the robotic arm 2 is always close to the patient also when the operating table 8 is moved in the operating room 12 or if the height of the operating table 8 is adjusted to the needs of the operating staff. Furthermore, the operating table 8 provides a stable basis for the robotic arm 2 in this embodiment. Nonetheless, the robotic arm 2 may also be placed on a separate basis, for instance on a roller, so that it can be moved independently from the operating table 8.

The robotic arm 2 in Fig. 1 is made from seven segments 3 connected by six joints 4 driven by motors 5. In Fig. 1, some of the joints 4 are illustrated as hinge joints, others are rotational joints. Also the number of segments 3 is not particularly limited and may be adapted in accordance with the requirements of the specific application. Typically, the robotic arm may have six or seven degrees of freedom. The segments 3 are driven by motors 5. Typically, individual motors 5 are placed in each joint 4 for driving the movement of the specific joint 4. The motors 5 may also be torque-controlled motors 5 and thereby include or serve as torque sensors, measuring the torque acting on each of the segments. In addition, the joints 4 may comprise position encoders 24 that are able to measure the current position of each joint 4 in real time or near real time. Alternatively or additionally, the current pose of the robotic arm 2 may be determined by a navigation system 25 described below. The torque controlled motors 5 may collectively act as force determination means 23 that are capable of determining a force vector 41 representing a magnitude and a direction of a force applied by the robotic arm 2 on an external object. In order, to distinguish the gravitational pull on the robotic arm 2 from other forces (like counterforces when the robotic arm 2 presses against a structure, forces from additional equipment put on the robotic arm 2 or forces applied by the staff in order to reposition the robotic arm 2, etc.) the force determination means 23 can comprise a kinematic model of the robotic arm 2 that is able to deduce the expected torque on each joint 4 expected from gravitation. The remaining torque can then be identified as caused by other external influences.

The robotic arm 2 acts on the anatomical structure 20 using its end-effector 6. The end-effector 6 is typically attached to the segment 3 that is furthest from the base of the robotic arm 2. The end-effector 6 can itself form a tool 7 that is used to act on an anatomical structure 20 and perform a task or the end-effector 6 may be a type of universal adapter that can hold a variety of different tools 7. For supporting different tools 7 the end-effector 6 may comprise connectors to provide electric, pneumatic or any other kinds of connections that a specific tool 7 may require.

The system 1 may further comprise a navigation system 25. The navigation system 25 may for instance be an optical navigation system including the components shown in Fig. 1. Such a system 25 is able to track the position of specific elements like trackers in space using for instance infrared light. The navigation system 25 comprises a stand 10 that may carry sensors 9, like cameras, tracking lasers or other components of the navigation system 25. The navigation system 25 is also connected to a processing unit 26 or comprises the processing unit 26 which is able to generate a reference coordinate system and determine the position of objects like trackers in this reference coordinate system. The processing unit may further be connected to the robotic arm 2 and supply data to the control unit 22 of the robotic arm 2. The reference coordinate system can be generated in relation to the position of the stand 10 of the navigation system 25. This means that any movement of the stand 10 would result in a change of a movement of the reference coordinate system. Typically, navigation during operations is based on a relative positions between different objects tracked by the navigation system 25. Therefore, such a change of the reference coordinate system does not interfere with the functionality of the navigation. Thus, the stand 10 of the navigation system 25 does not need to be securely fixed in position but may be placed on a roller as shown in Fig. 1.

The system in Fig. 1 also comprises a display 11. The display 11 may be connected by wire or wirelessly to the robotic arm 2 and/or the navigation system 25. The display 11 can show relevant information to the surgical staff. For instance, the display 11 may show the objects tracked by the navigation system 25. It may alternatively illustrate the actions and the forces applied by the robotic arm 2. This can also be combined with an anatomical model, thus illustrating the mechanical load and stress applied by the robotic arm 2 on a specific anatomical structure 20. The display 11 may also show FEM simulations of the anatomical structure 20 and the tool 7 held by the robotic arm 2. Display 11 can also be used to show the state of the force-controlled tracking, i.e., if contact is made to the anatomical structure 20, if the applied force is within the predefined ranges and/or by how much the position of the tool 7 deviates from the predefined trajectory 31 etc.

Fig. 2 shows a schematic drawing of the components of the system 1 for positioning a tool 7 on an anatomical structure 20. One component of the system 1 is the robotic arm 2. It comprises the mechanical components, i.e., the segments 3 connected by joints 4, which are driven by motors 5. The end-effector 6 is attached to one of the joints, typically the one furthest from the base of the robotic arm 2. These mechanical parts are able to hold and position a tool 7. The interaction between the robotic arm 2 and the tool 7 is illustrated by a dashed line in Fig. 2. Note that the end-effector 6 and the tool 7 do not have to be separate but can be one single part. The robotic arm 2 further comprises a control unit 22, which is configured to control the movement of the robotic arm 2 and receive data from units internal and external to the robotic arm 2. Examples of units included in the robotic arm 2 are the force determination means 23 and the position encoders 24. Both can be implemented within the mechanical structure of the robotic arm 2 but may comprise additional hardware and software, which also can be included on ECUs within the robotic arm 2. The force determination means 23 measures the force applied to the anatomical structure 20 by the robotic arm 2. As described above, this may involve the use of a kinematic model of the robotic arm 2. The position encoders 24 generate data on the current pose of the robotic arm 2. This may be based on measurements within the joints 4 of the robotic arm 2.

Another component of system 1, is the navigation system 25. The navigation system 25 may include mechanical components like the stand 8. It also comprises sensors 7 for tracking the position of objects in the operating room 12. In order to process the generated data and generate the reference coordinate system, the navigation system 25 comprises the processing unit 26.

As illustrated in Fig. 2, the tool 7 can be placed on the anatomical structure by the robotic arm 2 using its end-effector 6. A target tracker 21 may be placed on the anatomical structure 20 in proximity to the tool 7. The tracker 21 is located by the navigations system 25 using its sensors 9. The control unit 22 of the robotic arm 2 receives data from the robotic arm 2 itself (like from force determination means 23 and position encoder 24) and from the processing unit 26 of the navigation system 25. The connection between the processing unit 26 and the controller 22 is also illustrated by a dashed line. Through this interface data may be exchanged in either direction. In particular, the navigation system 25 may receive data from the position encoders of the robotic arm 2 via the controller 22.

As described above, display 11 can be used for displaying any information related to the navigation or force determination or the state of the robotic arm 2. It may therefore be part of the navigation system 25 or the robotic arm 2. In Fig. 2, the display 11 is shown as a separate part receiving and displaying information from both the control unit 22 of the robotic arm 2 and from the processing unit 26 of the navigation system 25.

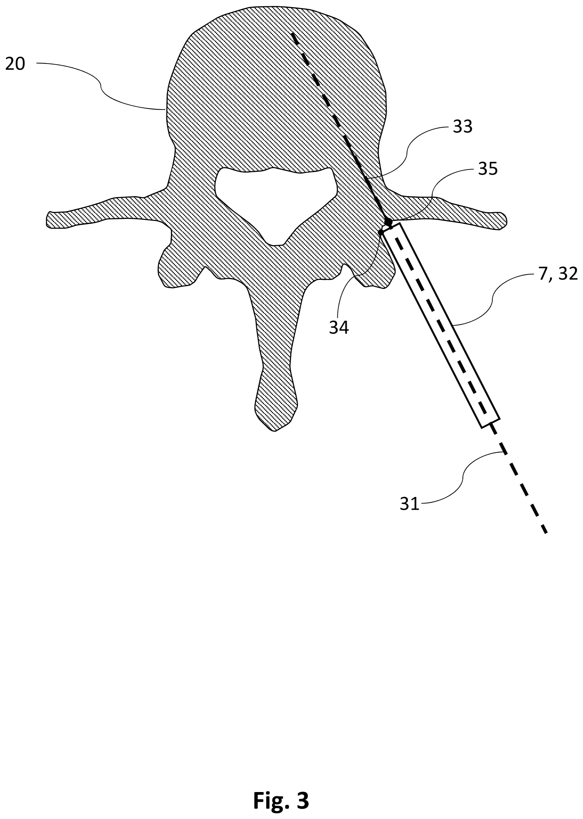

Fig. 3 shows a human vertebra 20 as an example of an anatomical structure 20 on which a tool 7 can be positioned. During spine surgery, screws often need to be positioned precisely in the vertebra 20. Correct positioning is particularly crucial in order to prevent damage to neighboring nerves or other sensitive structures. Before inserting a screw, a pilot hole 33 is drilled along the planned axis of the screw. For aligning the drill, a drill guide 32 may be used as the tool 7. A drill guide 32 is essentially a tube, which aligns a drill, which may be a handheld drill, when the drill is inserted in the drill guide. Additionally, the drill guide 32 may adjust the depth of the drilled hole by preventing the drill from being inserted further than the predefined maximal depth. The drill guide 32 may also be used subsequently for guiding a K-wire into the drilled hole and for inserting the screw. In Fig. 3 the drill guide 32 is illustrated by a rectangle.

The pilot hole 33 (illustrated by solid thin line in Fig. 3) is intended to be drilled along a predefined trajectory 31 which is illustrated by a wide dashed line in Fig. 3. The predefined trajectory 31 may be determined through medical imaging, such as CT scans. It can then be parametrized relative to the position of the tracker 21 that is fixed to the anatomical target 20. As described above, a tracker 21 is typically screwed to a vertebra that is two to three vertebrae apart from the target vertebra 20, into which the pilot hole 33 is drilled. This ensures that the relative position between the target vertebra 20 and the tracker 21 can only vary by a small amount as a result of a bending of the spine. All kinds of changes of the shape of the anatomical structure 20 may be referred to as deformations, this may involve a bend in the structure, an indentation of the surface of the structure or the like. Such deformations can be caused by the force applied by the tool 7 held by the robotic arm 2 or by other tools like a handheld drill. A deformation of the anatomical structure 20 can cause a deviation between the intended positioning of the pilot hole 33 and the end result when the drill is only guided relative to the target tracker. For an optimal result, the drill guide 32 should therefore follow the target structure 20 during the drilling process.

In order to guide the drill along the predefined trajectory 31, the drill guide 32 needs to be placed precisely along the predefined trajectory 31. Before making contact with the target vertebra 20 or any other part of the body, the movement of drill guide 32 is controlled in accordance with the position acquired by the navigation system 25. As described above, the predefined trajectory 31 is defined in relation to the target tracker 21, which is located by the navigation system 25. The position of the tool 7 may be deduced from a tracker placed on the tool 7 and located by the navigation system 25 and/or by using position encoders 24 in the robotic arm 2 that holds the tool 7. The robotic arm 2 can then perform a movement so that the drill guide 32 is placed along the predefined trajectory 31 and is moved along this trajectory 31 towards the intended drill point on the vertebra 20. During this movement, the controller of the robotic arm 2 monitors whether contact is made with the vertebra 20. Notably, contact is not necessarily made along the predefined trajectory 31. While an intended point of contact 35 may be directly on the predefined trajectory 31, the shape of the bone in combination with the shape of the drill guide 32 may result in an actual point of contact 34 that is not directly on the predefined trajectory 31.

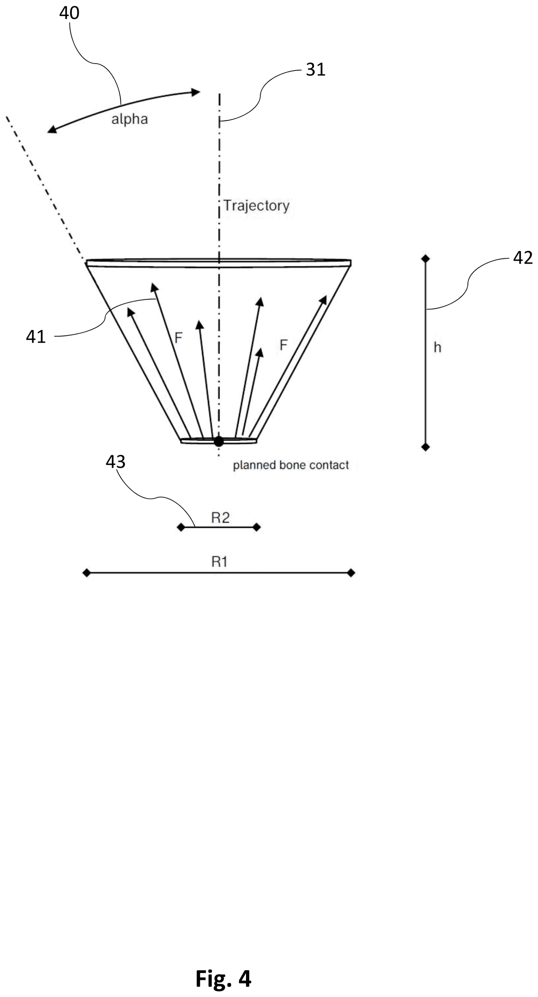

Once contact is made, the controller 22 may switch to a force-controlled positioning mode. In this mode, the robotic arm 2 is controlled to mimic the action of a surgeon that holds a tool 7 in place based on tactile response rather than a detected position in space. Fig. 4 illustrates the concept of force control based on acceptable force ranges resulting in a force cone.

The robotic arm 2 can determine the force applied on the anatomical structure 20 using the force determination means 23. The applied force 41 is a vector specifying the magnitude of the applied force and its direction. The robotic arm 2 also has the ability to control the applied force 41 and thereby balance the tool 7 on the target bone (anatomical structure 20). When for instance the magnitude is decreasing the robotic arm 2 can apply a stronger force. This may involve the vertebra 20 giving way to the applied force 41 by a certain amount until the counterforce matches the applied force 41 by the robotic arm 2. Likewise, if the forces increased again, for example due to inhalation and the associated repositioning of the target, the robotic arm 2 can move back accordingly in order to reduce the applied force 41. The robotic arm 2 can also change the direction in which the force is applied, this may but not necessarily involves pivoting of the tool 7.

The method involves adjusting the force so that the magnitude is within a predefined range 42 and the direction is within a predefined range of directions 40. The predefined range of directions 40 may for instance define a cone shape in space. The force cone is placed in the planned contact point between the tool 7 and the bone 20. The force cone is a kind of tolerance field of force vectors that are to be provided by the robotic arm 2. The range of predefined directions 40 can be defined by any direction forming an angle of less than an angle alpha with the predefined trajectory 31. This maximal angle of deviation from the predefined trajectory 31 is illustrated as alpha in Fig. 4.

The applied force 41 is also limited to not exceed an upper boundary. This boundary can be illustrated by the height of the cone denoted h in Fig. 3. In general, the predefined range 42 of the magnitude can be any range having an upper and lower boundary and this boundary may or may not depend on the current angle between the force vector and the predefined trajectory 31. For instance, the control algorithm may allow a stronger force to be applied (i.e. use a larger upper boundary of the force range) when the angle between the force and the predefined directory 31 is larger. A lower boundary of the force may be chosen so that the tool is always ensured to stay in firm contact with the target 20. In Fig. 4 a lower boundary is not illustrated.

As illustrated in Fig. 4, the force cone can also be a conical frustum, when the tolerance of the point of contact 34 is taken into account. In Fig. 4, a conical frustum is shown which has a smaller area on the side facing the bone with a diameter of R2. This smaller area represents the tolerance of the displacement 43 of the contact point from the initial contact point or from the predefined trajectory 31. This position tolerance 43 may for instance be chosen as the core diameter of the tool 7 guided and positioned by the robotic arm 2 and/or it may be chosen in accordance with an anatomical model including the flexibility and elasticity of the anatomical target structure 20. This additional condition, that the point at which the force is applied should not deviate more than a predefined maximal distance from the predefined trajectory 31 means that if the target 20, i.e. if the contact point leaves area R1, the allowed force vectors approach zero and the robotic arm 2 moves again in order to meet the force specifications taking into account position and magnitude of the force.

The allowed range of directions can be parametrized by an allowed maximal angle alpha 40 or the ratio of the two diameters of upper and lower surface of the frustum R2/R1. In each case, the height is related to the upper boundary of the force range.

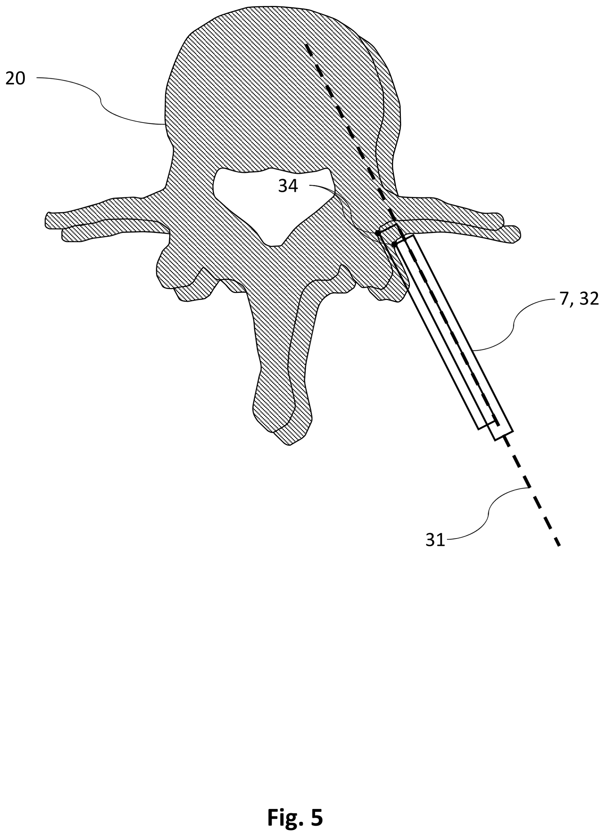

Fig. 5 illustrates the effect of a force controlled positioning of the tool 7. After making contact with the bone 20, the drill guide 32 follows the movement of the bone 20, which is illustrated in Fig. 5 by the overlay of two different positions of bone 20 and drill guide 32. The contact point 34 stays in place on the bone 20 even when the bone 20 as a whole moves. The drill guide 32 follows this movement by keeping the applied force within the predefined ranges as illustrated by the force cone. This also allows deviating from the predefined trajectory 31 by a certain amount. In the example of Fig. 5, the movement is a translational movement that does not involve rotation of the bone 20. As outlined below, the movement of the bone 20 may also involve rotation.

In order to enable such a force-based control, slipping of the tool on the anatomical structure 20 is to be avoided. In the example of a drill guide 32 being positioned on the bone 20, slipping may be avoided by forming the contact point or points of the drill guide 32 as sharp edges, spikes or using a tooth-shaped edge.

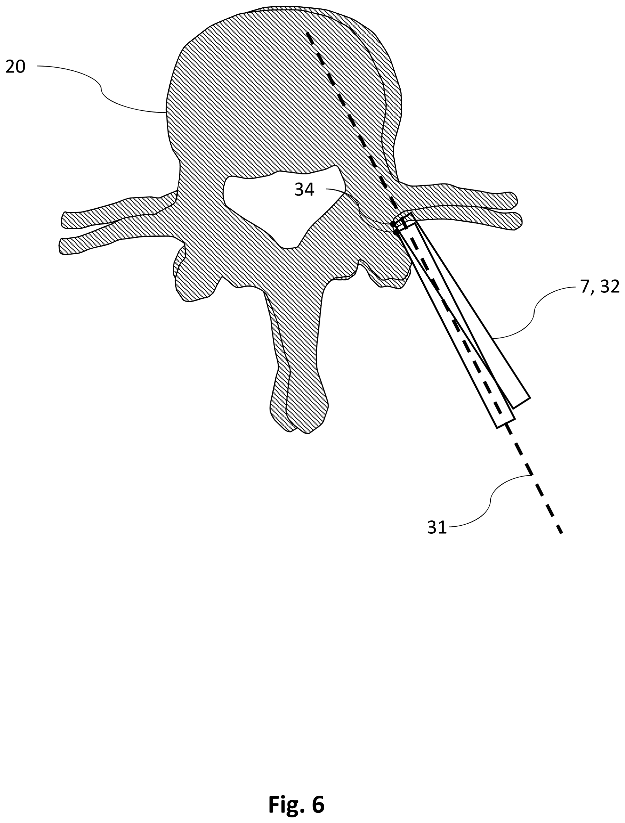

Fig. 6 is largely analogous to Fig. 5. It illustrates the effect of a force controlled positioning of the tool 7 in the case when the movement of the bone 20 involves translation and rotation. A rotational component of the movement of the bone 20 may be caused by two eccentric forces acting on the bone 20. A rotation may cause the counterforce applied by the bone 20 to the tool 7 (which is equivalent to the force applied by the tool 7 to the bone 20) to change its direction and thus apply a torque to the tool 7. The change in the direction of the force 41 is also measured by the force determination means 23. When the force direction is outside of the predefined range of directions, i.e. outside of the force cone, the tool adjust the position of the tool in order to keep the force within the predefined ranges. As a result, the tool 7 follows the movement of the bone 20, also when the movement involves rotation of the bone 20.

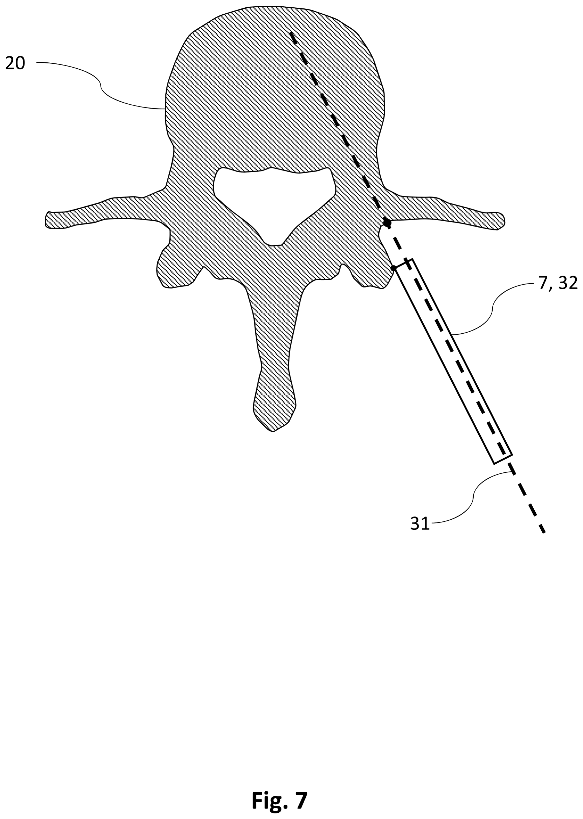

Fig. 7 illustrates the case in which the tool 7 does not make contact with the anatomical structure 20 in the expected position. During movement of the tool along the predefined trajectory 31, some deviations may occur. This can be due to the inaccuracies of the tracking, sudden movements of the target 20 or an external force applied on the robotic arm 2. As outlined above, the robotic arm 2 is generally configured to switch into force control mode when a contact between the tool 7 and an anatomical structure 20 is detected. In order to ensure that the contact is made at the intended position, the robotic arm 2 may perform an optional check before switching into force-controlled tracking mode. Accordingly, the controller may be configured to, when it is determined that the tool 7 is in contact with the anatomical structure 20, compare the current position of the tool 7 with a position in which the contact is expected to take place. If it is determined that the current position, in which contact was detected, differs by more than a predefined maximal deviation from the expected position of the first contact, the controller 22 can be configured to pull the tool 7 back and to restart moving the tool 7 along the predefined trajectory 31. Alternatively, the controller 22 may abort the procedure and/or sound an alarm or signal in some other manner that the positioning of the tool 7 has not been successful. The predefined maximal deviation may be chosen in accordance with the accuracy requirements of a given application and the shape of the anatomical structure 20 and the tool 7. Larger deviations from the expected point of first contact may be acceptable for larger tools 7. The predefined maximal deviation can also depend on the direction of the deviation. For instance, a larger or smaller deviation may be acceptable the predefined trajectory 31 compared to a deviation perpendicular to the predefined trajectory 31.

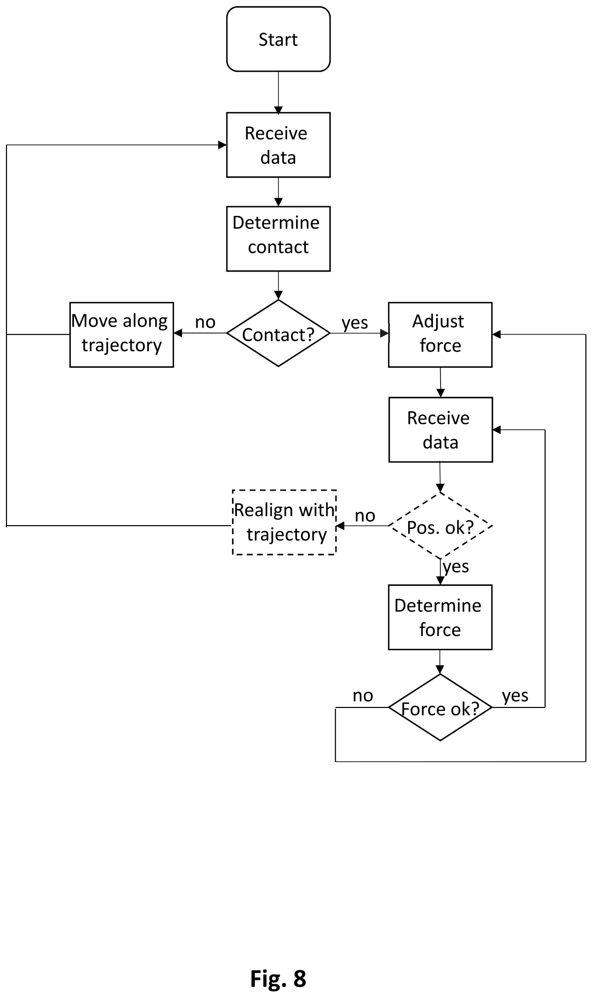

Fig. 8 is a flow chart of a method for positioning a tool 7 on an anatomical structure 20. The method may be carried out by a controller 22 of a robotic arm 2. Nonetheless, some of the steps may involve other components of the system 1 for positioning a tool 7 described above.

The method starts at the top of Fig. 8. The control unit 22 of the robotic arm 2 starts by receiving data that is indicative of the state of the robotic arm 2 and/or data that represents the position of objects like the target tracker 21 as determined by the navigation system 25. From the received data, it is determined whether the tool 7 is in contact with the anatomical structure 20. If contact has not been made, the method proceeds to move the tool 7 along the predefined trajectory 31. The method then return to the initial step of receiving data.

If it is determined that the tool 7 held be the end-effector 6 of the robotic arm 2 is in contact with the anatomical structure 20, the method switches to force-control and adjusts the force 41 applied by the tool 7 to the anatomical structure, Adjusting the force 41 includes controlling the motors 5 such that the expected amount of force applied to the anatomical structure 20 matches the intended applied force, which is typically the center value of the acceptable ranges of force magnitude and force direction. The method proceeds with receiving the data from the navigation system 25 and the robotic arm 2. The received data indicates the positions of the target tracker 21 and the tool 7 and the force 41 applied by the tool 7 to the anatomical structure 20.

Subsequent to receiving data in the force control mode, Fig. 8 includes an optional step of checking if the current position of the tool 7 is as intended, in particular it is checked whether the position of the end-effector 6 relative to the target tracker 21 does not deviate more than a predefined maximal distance 43 from the predefined trajectory 31. If this check is negative, the system 1 determines that the tool 7 must have lost its intended point of contact with the anatomical structure 20. Thus, the method proceeds to realign the tool 7 with the predefined trajectory 31. This may be done by first pulling the tool 7 back and then approaching the anatomical structure 20 again. Alternatively, it may be done by moving the tool 7 along the anatomical structure 20. After the realignment, the method proceeds with the initial step of the method, i.e., before determining contact.

If it is determined that the current position of the tool 7 does not deviate more than the predefined maximal distance 43 from the predefined trajectory 31 or if the optional steps are not included, the controller 22 determines the applied force 41 from the received data. It is then evaluated whether the force 41 is in line with the predefined range of directions 40 and the predefined force range 42. If the force 41 is within the predefined ranges, the method proceeds to repeatedly receive data and control the applied force 41. If it is determined that the force 41 falls outside of at least one of the predefined ranges, the force 41 is adjusted in order to match again with the intended force.

The method shown in Fig. 8 may be simplified. In particular, further steps may be included or the order of the steps may be changed. Furthermore, decisions shown as binary in the flow chart may be more complex in an actual implementation. For instance, when evaluating whether the applied force 41 is in line with the acceptance ranges, the method may also adjust the applied force 41 when the force is still within the acceptable ranges but is approaching a boundary. In this way, the force may be controlled to stay within the acceptable ranges without ever leaving them.

### List of reference signs (part of the description)

- 1: System for positioning a tool
- 2: Robotic arm
- 3: Segments of the robotic arm
- 4: Joints
- 5: Motors
- 6: End-effector
- 7: Tool
- 8: Operating table
- 9: Sensors of navigation system
- 10: Stand for sensors of navigation system
- 11: Display
- 12: Operating room
- 20: Anatomical structure
- 21: Target tracker
- 22: Controller of the robotic arm
- 23: Force determination means
- 24: Position encoders
- 25: Navigation system
- 26: Processing unit of navigation system
- 31: Predefined trajectory
- 32: Drill guide
- 33: Pilot hole
- 34: Contact point
- 35: Expected contact point
- 40: Predefined range of directions
- 41: Applied force
- 42: Predefined force range
- 43: Predefined maximal distance from predefined trajectory

## Claims

1. A system (1) for positioning a tool (7) relative to an anatomical structure (20), the system (1) comprising
a robotic arm (2) comprising
a plurality of segments (3) driven by motors (5);
an end-effector (6) attached to the robotic arm (2) and configured to hold the tool (7), wherein the robotic arm (2) is configured to position the tool (7) and to adjust a force (41) applied to the tool (7) by driving the motors (5); and
at least one force determination means (23) to determine a force vector representing a magnitude and a direction of the force (41) applied by the tool (7) to the anatomical structure (20);
a target tracker (21) configured to be placed on the anatomical structure (20);
a navigation system (25) configured to generate a reference coordinate system and to determine positions of the target tracker (21) and the tool (7) in the reference coordinate system;
a controller (22) configured to
receive data indicating the positions of the target tracker (21) and the tool (7) from the navigation system (25) and data indicating the force (41) applied by the tool (7) to the anatomical structure (20);
determine from the received data whether the tool (7) is in contact with the anatomical structure (20); and
control the robotic arm (2) in accordance with the received data and the determination of a contact or no contact with the anatomical structure (20);
wherein the controller (22) is configured to control the robotic arm (2) to perform the following steps
when it is determined that the tool (7) is not in contact with the anatomical structure (20), moving the tool (7) along a predefined trajectory (31) relative to the position of the target tracker (21) until it is determined that the tool (7) is in contact with the anatomical structure (20);
after determining that the tool (7) is in contact with the anatomical structure (20), adjusting the force (41) applied to the tool (7) such that the magnitude of the force (41) is within a predefined force range (42) and the direction of the applied force (41) is within a predefined range of directions (40),
wherein adjusting the force (41) applied to the tool (7) involves adjusting the position of the tool (7) and following the anatomical structure (20) when the anatomical structure (20) moves in any direction.

2. The system according to claim 1, wherein the controller (22) is further configured to, after determining that the tool (7) is in contact with the anatomical structure (20), adjust the position of the tool (7) using the robotic arm (2) such that the position of the end-effector (6) relative to the target tracker (21) does not deviate more than a predefined maximal distance (43) from the predefined trajectory (31).

3. The system according to one of claims 1 or 2, wherein the controller (22) is configured to determine whether the tool (7) is in contact with the anatomical structure (20) based on the data indicating the force (41) applied by the tool (7) to the anatomical structure (20).

4. The system according to one of the previous claims, wherein the controller (22) is configured to determine whether the tool (7) is in contact with the anatomical structure (20) by determining if the robotic arm (2) can apply a force to the tool (7) without moving the tool (7) in the corresponding direction.

5. The system according to claim 4, wherein the controller (22) is further configured to
upon determining that the tool (7) is in contact with the anatomical structure (20), record an initial point of contact which corresponds to the position of the tool (7) relative to the target tracker (21) at a moment of first contact;
determine a deformation of the anatomical structure (20) by determining that the by adjusting the force (41) applied to the tool (7), the position of the end-effector (6) relative to the target tracker (21) has changed from the initial point of contact.

6. The system according to claim 5, wherein the controller (22) is further configured to determine a flexibility and/or elasticity of the anatomical structure (20) based on the force (41) applied to the anatomical structure (20) and the deformation of the anatomical structure (20).

7. The system according to one of the previous claims, wherein the predefined trajectory (31), the force range (42) and the range of directions (40) can be set by a user of the system (1).

8. A method of determining control instructions for a robotic arm (2),
wherein the robotic arm (2) comprises a plurality of segments (3) connected by joints (4) and driven by motors (5), and the control instructions comprise computer-readable commands for the robotic arm (2) to adjust a pose of the robotic arm (2) and/or a torque generated by at least one of the motors (5) acting in at least one of the joints (4);
the method comprising:
receiving data indicating the position of a target tracker (21) and a position of a tool (7) held by the robotic arm (2);
receiving, from a force determination means (23) of the robotic arm (2), data indicating a force (41), wherein the data indicates a force vector representing a magnitude and a direction of a force (41) acting on the tool (7);
determining from the received data whether the tool (7) is in contact with an object;
determining control instructions for the robotic arm (2) based on the received data and the determination of a contact or no contact with the object;
wherein determining control instructions for the robotic arm (2) comprises
when it is determined that the tool (7) is not in contact with the object, generating control instructions to adjust the pose of the robotic arm (2) to move the tool (7) along a predefined trajectory (31) relative to the position of the target tracker (21) until it is determined that the tool (7) is in contact with the object; and
after determining that the tool (7) is in contact with the object, generating control instructions to adjust the torque by at least one of the motors (5) acting in at least one of the joints (4) such that the magnitude of the force (41) is within a predefined force range (42) and the direction of the force (41) is within a predefined range of directions (40) and such that the tool follows the object when the object moves in any direction.

9. The method according to claim 8, further comprising, after determining that the tool (7) is in contact with the object, generating control instructions to adjust the pose of the robotic arm (2) such that the position of the tool (7) relative to the target tracker (21) does not deviate more than a predefined maximal amount (43) from the predefined trajectory (31).

10. The method according to one of claims 8 or 9, wherein determining whether the tool (7) is in contact with the object is based on the data indicating the force (41) acting on the tool (7).

11. The method according to one of claims 8 to 10, wherein determining whether the tool (7) is in contact with the object involves determining if the robotic arm (2) can apply a force to the tool (7) without moving the tool (7) in the corresponding direction.

12. The method according to one of claims 8 to 11, wherein the method further comprises receiving a user input and setting the predefined trajectory (31), the predefined force range (42) and the predefined range of directions (40) in accordance with the user input.

13. A robotic arm (2) for positioning a tool (7) relative to an anatomical structure (20), wherein the robotic arm (2) comprises
a plurality of segments (3) driven by motors (5);
an end-effector (6) attached to the robotic arm (2) and configured to hold the tool (7), wherein the robotic arm (2) is configured to position the tool (7) and to adjust a force (41) applied to the tool (7) by driving the motors (5); and
at least one force determination means (23) to determine a force vector representing a magnitude and a direction of a force (41) applied by the tool (7) to the anatomical structure (20);
a controller (22) configured to
receive data indicating the positions of a target tracker (21) and the tool (7) and data indicating the force (41) applied by the tool (7) to the anatomical structure (20);
determine from the received data whether the tool (7) is in contact with the anatomical structure (20); and
control the robotic arm (2) in accordance with the received data and the determination of a contact or no contact with the anatomical structure (20);
wherein the controller (22) is configured to control the robotic arm (2) to perform the following steps
when it is determined that the tool (7) is not in contact with the anatomical structure (20), moving the tool (7) along a predefined trajectory (31) relative to the position of the target tracker (21) until it is determined that the tool (7) is in contact with the anatomical structure (20);
after determining that the tool (7) is in contact with the anatomical structure (20), adjusting the force (41) applied to the tool (7) such that the magnitude of the force (41) is within a predefined force range (42) and the direction of the applied force (41) is within a predefined range of directions (40),
wherein adjusting the force (41) applied to the tool (7) involves adjusting the position of the tool (7) and following the anatomical structure (20) when the anatomical structure (20) moves in any direction.

14. A computer program product comprising instructions to cause the system (1) of one of claims 1 to 7 to execute the steps of the method of one of claims 8 to 12.
